# EUROPÄISCHE PATENTSCHRIFT

(11) **EP 2 981 306 B1**
(45) Veröffentlichungstag und Bekanntmachung des Hinweises auf die Patenterteilung: **12.08.2020**
(21) Anmeldenummer: 14715825.7
(22) Anmeldetag: 27.03.2014
(51) Int. Cl.: A61M 1/06, F16K 37/00, F16K 15/03

(54) **VORRICHTUNG MIT EINEM DURCHFLUSSKANAL**
DEVICE HAVING A FLOW CHANNEL
DISPOSITIF DOTÉ D'UN CONDUIT D'ÉCOULEMENT

(30) Priorität: 02.04.2013 CH 698132013
(43) Veröffentlichungstag der Anmeldung: 10.02.2016
(73) Patentinhaber: Medela Holding AG, 6340 Baar (CH)
(72) Erfinder: FELBER, Armin, 6003 Luzern (CH)
(74) Vertreter: Clerc, Natalia
(86) Internationale Anmeldenummer: PCT/CH2014/000041
(87) Internationale Veröffentlichungsnummer: WO 2014/161099

(56) Entgegenhaltungen:
- WO-A1-98/36245
- WO-A1-2012/081228
- DE-A1- 3 344 196
- DE-A1-102008 062 532
- US-A1- 2011 301 532

## Beschreibung

### TECHNISCHES GEBIET

Die vorliegende Erfindung betrifft eine Vorrichtung mit einem Durchflusskanal gemäss Oberbegriff des Patentanspruchs 1. In einem bevorzugten Anwendungsbereich ist diese Vorrichtung eine Brusthaube einer Brustpumpeneinheit zum Abpumpen von menschlicher Muttermilch bzw. ein Adapter zwischen Brusthaube und Milchsammelbehälter.

### STAND DER TECHNIK

Zum Abpumpen von menschlicher Muttermilch werden motorisch oder manuell betriebene Brustpumpeneinheiten eingesetzt. Diese Brustpumpeneinheit weist eine oder zwei Brusthauben auf, welche auf die Mutterbrust aufgesetzt werden und die Brustwarze umfassen. Mittels einer Vakuumpumpe wird ein Unterdruck in der Brusthaube angelegt, welcher vorzugsweise zyklisch variiert. Eine Milchflasche ist über einen Adapter oder direkt an die Brusthaube angeschraubt, damit abgepumpte Milch direkt in einen Auffangbehälter fliessen kann. Derartige Brustpumpeneinheit sind hinlänglich bekannt, beispielsweise aus US 6 497 677, US 4 964 851, US 6 547 756 und US 6 257 847.

Um das Totvolumen in der Brusthaube zu begrenzen, weist die Brusthaube oder der Adapter ein Rückschlagventil auf, welches einen Durchgang zum Innenraum der Milchflasche öffnet. Das Rückschlagventil ist beispielsweise ein Membranventil mit einer Ventilmembran, wie sie in einigen der oben genannten Patentpublikationen sowie auch in US 4 929 229, US 8 070 716 und US 2011/0301532 offenbart ist.

EP 1 430 918 beschreibt ein System zur Detektion des Milcheinschusses. Hierfür wird ein Milchsammelbehälter während des Abpumpens auf eine Waage gestellt, um die abgepumpte Milchmenge als Funktion der Zeit zu bestimmen.

Des Weiteren ist aus der noch unveröffentlichten internationalen Patentanmeldung PCT/CH2012/000222 eine Vakuumpumpe zur Verwendung in einer Brustpumpeneinheit bekannt, welche eine Medienseparationsmembran aufweist. Diese Membran dient zudem als Sensormembran zur Bestimmung des erzeugten Unterdrucks. Hierfür weist die Membran eine vorstehende Fahne auf, deren Position optisch detektiert wird.

US 2012/0022451 offenbart eine Pumpeinheit für eine enterale Ernährungsvorrichtung. Diese Pumpeinheit weist eine durch eine Membran getrennte Doppelkammer auf. Die Membran ist in einer Ausführungsform mit einem Anzeigestab versehen, welcher sich entsprechend der Bewegung der Membran anhebt und absenkt und als Mass des in einer Kammer befindlichen Fluids dient.

DE 10 2008 062 532 A1 offenbart ein Absperrelement mit einer Rückschlagklappe, welche mit einem Permanentmagneten versehen ist. Dieser Permanentmagnet wird von einem Sensor erfasst, so dass die Position der Klappe bestimmt werden kann.

DE 33 44 196 A1 zeigt ebenfalls eine Rückschlagklappe mit darauf angeordnetem Magneten.

WO 2012/081228 A1 offenbart ein Flüssigkeitsregelventil mit einem Ventilkörper, einer Antriebswelle und einem Antrieb. Der Ventilkörper ist mit einer Fahne versehen, die in eine Lichtschranke gelangt, um die Position des Ventilkörpers zu erkennen.

### DARSTELLUNG DER ERFINDUNG

Es ist eine Aufgabe der Erfindung, bereits während des Abpumpvorgangs eine Indikation für einen Fluidfluss, vorzugsweise ein Mass für die Menge des abgepumpten Fluids, zu erhalten.

Diese Aufgabe löst eine Vorrichtung mit den Merkmalen des Anspruchs 1.

Die erfindungsgemässe Vorrichtung weist einen Durchflusskanal für ein Fluid auf. Im Durchflusskanal ist ein Rückschlagventil angeordnet, welches es dem Fluid ermöglicht, den Durchflusskanal in einer ersten Richtung zu durchströmen und welches ein Durchströmen des Durchflusskanals in einer zur ersten Richtung entgegen gesetzten Richtung verhindert. Die Vorrichtung weist ferner einen Durchflussdetektor zur Detektion des Durchflusses des Fluids durch den Durchflusskanal auf, wobei der Durchflussdetektor eine Veränderung des Rückschlagventils detektiert. Diese detektierte Veränderung dient als Indikator für den Durchfluss.

Vorzugsweise ist der Durchflussdetektor ein Durchflussmesser zur Bestimmung des Durchflusses, wobei die detektierte Veränderung als Mass für den Durchfluss dient.

Die Vorrichtung ist in bevorzugten Ausführungsbeispielen Teil einer Brusthaube oder eines Adapters zwischen Brusthaube und Milchsammelbehälter. Das Fluid ist in diesem Fall die abgepumpte Milch. Der Fluidsammelbehälter der Milchbehälter.

Die erfindungsgemässe Vorrichtung lässt sich jedoch auch in anderen Bereichen einsetzen, beispielsweise in Drainagevorrichtungen, insbesondere bei der Thoraxdrainage oder der Wunddrainage. Das Fluid kann in diesem Fall ein abgesaugtes Sekret oder Luft sein. Die Vorrichtung ist in diesen Anwendungsbereichen vorzugsweise am oder im Bereich eines Fluidsammelbehälters angeordnet. Beispielsweise lässt sich ein Deckel eines Fluidsammelbehälters gemäss WO 2008/141471 oder WO 2007/085100 mit einem derartigen Rückschlagventil und Durchflussdetektor ausrüsten.

Fliesst ein Fluid durch das Ventil, so öffnet sich dieses und verändert sich dadurch in seiner Form oder Position im Raum. Dies ist somit ein detektierbarer Indikator für den Fluidfluss.

Fliesst viel Fluid durch das Ventil, d.h. ist die Durchflussmenge gross, so ist die Veränderung des Rückschlagventils relativ zu seiner Ruhelage ohne Durchfluss grösser als wenn die Durchflussmenge klein ist. D.h. das Rückschlagventil muss sich bei einer grösseren Fluidmenge mehr öffnen als bei einer kleineren Menge. Dadurch lässt sich auf einfache Weise ein Mass für den Durchfluss erhalten.

Dieses Mass lässt sich beispielsweise mit oder ohne weitere Berechnungen graphisch auf einem Display anzeigen, so dass beispielsweise die Mutter auf einfache Weise erkennen kann, ob die abgepumpte Milchmenge zu- oder abnimmt bzw. ob sie konstant bleibt. Gleiches gilt auch für andere Anwendungsbereiche.

Dieses Mass lässt sich auch mittels einer elektronischen Auswerteeinheit auswerten. Es lässt sich beispielsweise die Durchflussrate, d.h. die Durchflussmenge pro Zeit berechnen. Des Weiteren lässt sich die Durchflussmenge bzw. die gesamte abgepumpte Milchmenge berechnen. Ebenso lässt sich der Zeitpunkt des Milcheinschusses, d.h. der Zeitpunkt, an welchem die Milch maximal zu fliessen beginnt, bestimmen. Gleiches gilt auch für andere Anwendungsbereiche.

Der Durchflussdetektor kann die Veränderung des Rückschlagventils auf unterschiedliche Arten detektieren, optisch oder mechanisch. Erfindungsgemäss, weist der Durchflussdetektor einen optischen Detektor zur Detektion der Veränderung des Rückschlagventils auf. Der optische Detektor umfasst einen Lichtemitter und einen Lichtdetektor, wobei das Rückschlagventil im veränderten und/oder im unveränderten Zustand in einem Lichtpfad zwischen Lichtemitter und Lichtdetektor angeordnet ist. Vorzugsweise ist das Rückschlagventil so angeordnet, dass es mindestens im veränderten Zustand den Lichtpfad unterbricht.

Die Lage des Rückschlagventils lässt sich auf einfache Weise detektieren, wenn das Rückschlagventil eine Ventilmembran aufweist, wobei der Durchflussdetektor eine Auslenkung der Ventilmembran feststellt. Die Detektion ist vereinfacht, wenn die Ventilmembran eine Ventilklappe aufweist. Fliesst wenig Fluid, so wird die Membranklappe kaum ausgelenkt, bei einer grösseren Durchflussmenge entfernt sie sich weiter weg von der Ventilöffnung ihres Ventilsitzes. Der Weg ist umso grösser je grösser die Ventilmembran ist. Dies erhöht die Genauigkeit der Messung. Der Weg lässt sich auch vergrössern, indem die Ventilklappe in einem ersten Randbereich am Ventilsitz befestigt und an einem zweiten, dem ersten Randbereich gegenüber liegenden Randbereich relativ zum Ventilsitz bewegbar ist; d.h. die Ventilklappe ist azentrisch einseitig befestigt.

Erfindungsgemäss ist das Rückschlagventil, insbesondere die Ventilmembran, mit einem Schwert oder einer steifen Fahne versehen, deren Lage der Durchflussdetektor detektiert. Auch dies erleichtert die Detektion und weist zudem den Vorteil auf, dass die Funktionsweise des Rückschlagventils nicht beeinträchtigt ist. Vorzugsweise ist die Fahne auf der Ventilmembran, insbesondere senkrecht einer Grundfläche der Ventilmembran vorstehend, angeordnet. Dabei ist die Fahne vorzugsweise im Lichtpfad angeordnet. Im Falle einer einseitig befestigten Ventilklappe ist die Fahne vorzugsweise im der Befestigung gegenüberliegenden Randbereich angeordnet, um bei Bewegung der Membran einen möglichst maximalen Weg zurückzulegen.

Vorzugsweise ist die Ventilmembran plan ausgebildet, wobei sie vorzugsweise auf ihrer planen Grundfläche mit Noppen versehen ist, welche auf der dem Ventilsitz abgewandten Oberseite der Grundfläche angeordnet sind.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine Brustpumpeneinheit mit einer erfindungsgemässen Vorrichtung in teilweiser perspektivischer Darstellung;
- Figur 2: eine Ventilmembran der Vorrichtung gemäss Figur 1 in einer Ansicht von hinten;
- Figur 3: ein Ventilsitz der Vorrichtung gemäss Figur 1 in einer Ansicht von vorne, in gespiegelter Darstellung;
- Figur 4: die Ventilmembran gemäss Figur 2 in einer Ansicht von vorne;
- Figur 5: ein Ventilsitz der Vorrichtung in einer zweiten Ausführungsform;
- Figur 6: einen Längsschnitt durch einen Teil einer Brustpumpeneinheit mit der erfindungsgemässen Vorrichtung gemäss einem dritten Ausführungsbeispiel mit geschlossenem Rückschlagventil;
- Figur 7: einen Längsschnitt durch den Teil der Brustpumpeneinheit gemäss Figur 6 mit offenem Rückschlagventil;
- Figur 8: eine schematische Darstellung der erfindungsgemässen Vorrichtung in einer Seitenansicht und
- Figur 9: die Darstellung gemäss Figur 8 in einer Ansicht von vorne.

### BESCHREIBUNG BEVORZUGTER AUSFÜHRUNGSFORMEN

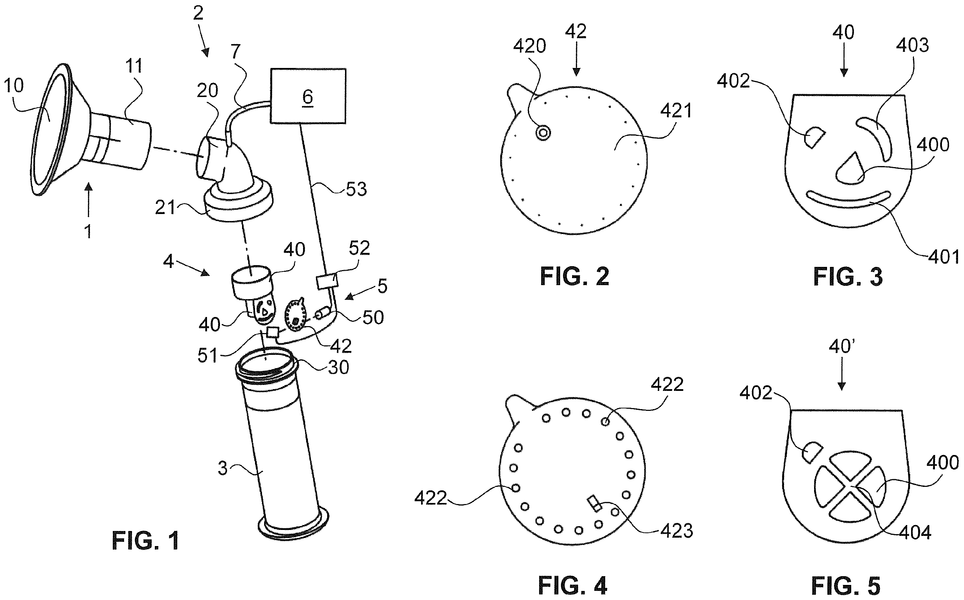

In Figur 1 ist eine Brustpumpeneinheit zum Abpumpen von menschlicher Muttermilch dargestellt. Sie weist eine Brusthaube 1 zum dichtenden Anlegen an die menschliche Mutterbrust, einen Milchsammelbehälter 3 zum Sammeln der abgepumpten Muttermilch und einen Adapter 2 zur lösbaren Verbindung der Brusthaube 1 mit dem Milchsammelbehälter 3 auf. Der Adapter 2 und die Brusthaube 1 können als getrennte Teile oder gemeinsam einstückig ausgebildet sein.

Die Brusthaube 1 kann eine im Stand der Technik bekannte Form aufweisen. Dargestellt ist eine übliche Form mit einem Trichter 10 zur Auflage an die Mutterbrust und einen daran angeformten Stutzen 11 zur Verbindung mit dem Adapter 2. Der Adapter 2 weist üblicherweise einen Stutzen 20 zur Aufnahme des Stutzens 11 der Brusthaube 1 auf sowie abgewinkelt dazu ein Gewindeteil 21 zur Verbindung mit einem Hals 30 des Milchsammelbehälters. Der Milchsammelbehälter kann steif, beispielsweise als Kunststoffflasche, oder flexibel, beispielsweise als Milchbeutel, ausgebildet sein.

Die Brustpumpeneinheit umfasst ferner eine Vakuumpumpe 6, welche über eine Saugleitung mit dem Adapter 2 bzw. der Brusthaube 1 verbunden ist. Die Vakuumpumpe 6 kann eine elektrische motorbetriebene Pumpe sein oder eine manuell betriebene Pumpe. Sie kann an der Brusthaube 1 bzw. am Adapter 2 angeordnet sein, so dass die Brustpumpeneinheit als portable Brustpumpe ausgebildet ist. Sie kann jedoch auch ein vom Adapter 2 getrenntes Gerät sein. Beispiele aller Varianten wurden bereits eingangs erwähnt.

Mittels der Vakuumpumpe 6 wird ein, vorzugsweise zyklisch variierender, Unterdruck in der Brusthaube 1 angelegt und die Muttermilch so abgepumpt. Um das Totvolumen zu begrenzen, ist in bekannter Weise ein Rückschlagventil 4 vorhanden. In diesem Beispiel ist es am Adapterteil 2 angeordnet. Das Rückschlagventil 4 weist einen Ventilsitz 40 mit mindestens einer Durchflussöffnung 400, 401, 403 auf, welche von einer Ventilmembran 42 verschlossen ist. Die Ventilmembran 42 ist vorzugsweise aus einem flexiblen Kunststoff, insbesondere Silikon, gefertigt.

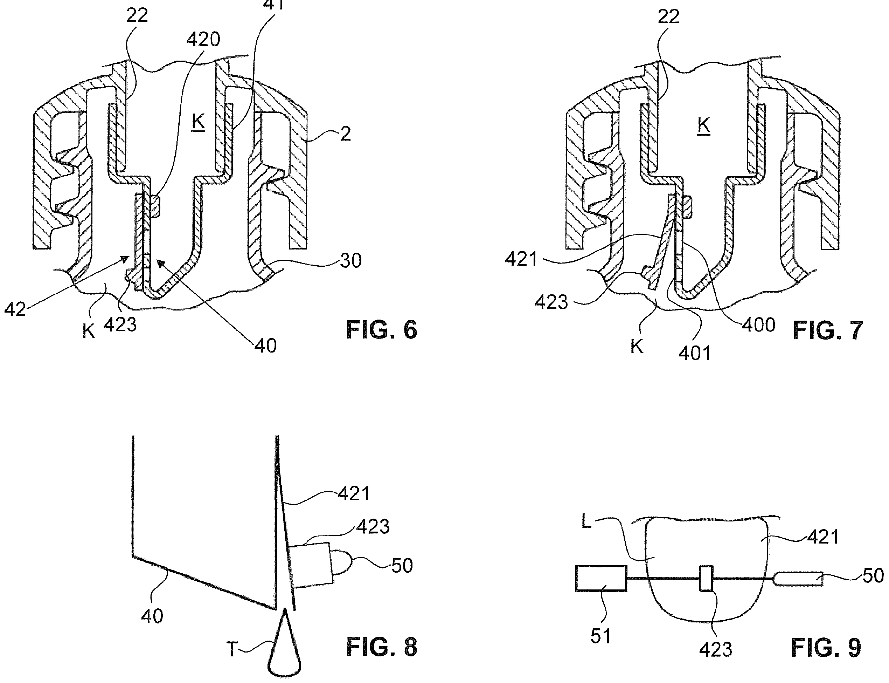

Der Ventilsitz 40 weist in diesem Beispiel einen Kragen 41 auf, welcher auf einen entsprechenden inneren Rohrabschnitt 22, welcher den Figuren 6 und 7 dargestellt ist, aufgesteckt ist. Der Ventilsitz 40 kann jedoch auch einstückig im Adapterteil 2 bzw. in der Brusthaube 1 angeformt sein.

Die Durchflussöffnungen 400, 401, 403 können verschiedenartig geformt sein. Sie können beispielsweise durch Stege 404 voneinander getrennt sein. Die Figuren 3 und 5 zeigen zwei mögliche Beispiele.

Die Ventilmembran 42 ist vorzugsweise einstückig ausgebildet. Sie ist vorzugsweise annähernd kreisrund oder oval geformt. Andere Formen sind jedoch ebenfalls möglich. Ihr Grundkörper 421 ist vorzugsweise flach und relativ dünn ausgebildet. Die annähernd plane Rückseite des Grundkörpers 421, d.h. die dem Ventilsitz zugewandten Seite, ist in Figur 2 dargestellt. Die Ventilmembran weist auf dieser Rückseite einen Noppen 420 auf, welcher in eine entsprechende Befestigungsöffnung 402 des Ventilsitzes 40 einsteckbar ist. Dieser Noppen 420 kann zentrisch auf dem Grundkörper 421 angeordnet sein. Vorzugsweise befindet er sich jedoch in einem Randbereich, so dass die Ventilmembran eine Ventilklappe bildet. Die Vorderseite der Ventilmembran, welche in Figur 4 dargestellt ist, kann plan ausgebildet sein oder sie kann, wie hier dargestellt, mehrere vorstehende Verstärkungsnoppen 422 aufweisen.

Erfindungsgemäss weist die Brustpumpeneinheit ferner einen Durchflussdetektor 5, vorzugsweise einen Durchflussmesser, auf. Dieser Durchflussdetektor 5 detektiert bzw. misst eine Veränderung des Rückschlagventils 4, wenn ein Fluid, hier Milch, durch das Rückschlagventil 4 strömt. Der Durchflussdetektor 5 ist in diesem Beispiel eine optische Detektionseinheit, welche einen Lichtemitter 50, beispielsweise eine Leuchtdiode oder ein Leuchtdiodenarray, und einen Lichtdetektor 51, beispielsweise eine Photodiode oder ein Photodiodenarray, umfasst.

Die Ventilmembran 42 weist eine der Vorderseite vorstehende Nase oder Fahne 423 auf, wie in Figur 4 erkennbar ist. Sie steht vorzugsweise senkrecht der Oberfläche des Grundkörpers 421 vor. Diese Fahne 423 ragt, wie in Figur 9 dargestellt ist, in einen Lichtpfad L, welcher durch Lichtemitter 50 und Lichtdetektor 51 gebildet ist. Eine Auswerteinheit 52, siehe Figur 1, ist mit dem Lichtemitter 50 und dem Lichtdetektor 51 verbunden. Sie kann beispielsweise im Bereich des Lichtdetektors 51 angeordnet sein oder sie kann im Gehäuse der Vakuumpumpe 6 integriert sein. Das Signal des Lichtdetektors 51 bzw. das Ergebnis der Auswerteeinheit 52 wird über eine Verbindungsleitung 53 zur Vakuumpumpe 6 geleitet. Die Übermittlung des Signals bzw. der Daten kann auch drahtlos erfolgen.

In Figur 6 ist die Ventilmembran 4 dargestellt, wenn keine Milch fliesst. Der Durchflusskanal K, gebildet durch den Innenraum der Brusthaube 1 bzw. des Adapters 2 und dem Innenraum des Milchsammelbehälters 3, ist durch das Rückschlagventil 4 unterbrochen.

Fliesst nun ein Fluid, hier ein Milchtropfen T, durch diesen Durchflusskanal K, so öffnet sich das Ventil 4, wie dies in den Figuren 7 und 8 dargestellt ist. Die Ventilmembran 42 wird um den Befestigungsnoppen 420 geschwenkt und die Fahne 423 wird ausgelenkt. Diese Auslenkung wird vom optischen Durchflussdetektor 5 detektiert.

Die Detektion kann auf unterschiedliche Weise erfolgen. Es kann eine einfache Auslenkung festgestellt werden. Beispielsweise befindet sich die Fahne 423 im nicht ausgelenkten Zustand ausserhalb des Lichtpfades L und es wird lediglich detektiert, dass sich die Fahne 423 bewegt hat. Somit wird lediglich detektiert, dass nun ein Fluid fliesst; es werden jedoch keine Rückschlüsse über die Menge gezogen. Dieses Ergebnis lässt sich optisch, akustisch oder auf andere Weise auf der Brustpumpeneinheit darstellen.

Es kann jedoch auch das Mass der Auslenkung bestimmt werden, indem festgestellt wird, wie weit die Fahne 423 in den Lichtpfad hineinragt. Das Mass der Auslenkung gibt Auskunft über die durch das Ventil geflossene Fluidmenge. Da das Vakuum üblicherweise zyklisch an die Mutterbrust angelegt wird, fliesst auch die Milch nicht gleichmässig. Es lässt sich die Milchmenge pro einzelnen Hub sowie die Milchmenge über einen vordefinierten Zeitraum bestimmen. Beides lässt sich beispielsweise graphisch und quantitativ auf einem Display der Brustpumpeneinheit optisch darstellen. Es hat sich gezeigt, dass sich im Falle der Verwendung mit einer Brusthaube das Ventil bereits während des Anlegens des Unterdrucks an die Brusthaube leicht öffnen kann, ohne dass bereits Milch fliesst. Diese Öffnung erfolgt üblicherweise zyklisch, analog zum Anstieg des Drucks in der Brusthaube. Diese zusätzliche Auslenkung lässt sich jedoch bei der Auswertung des Signals mitberücksichtigen und verfälscht dadurch das Ergebnis nicht.

Wie bereits erwähnt lässt sich dasselbe Prinzip der Durchflussdetektion und Durchflussmessung auch in anderen Bereichen, wie beispielsweise bei einer Drainagevorrichtung, einsetzen.

Die erfindungsgemässe Vorrichtung ermöglicht eine Durchflussdetektion mit einfachen Mitteln, indem ein vorhandenes Rückschlagventil als Anzeigemittel verwendet wird.

**BEZUGSZEICHENLISTE**

| | | | |
|---|---|---|---|
| 1 | Brusthaube | 420 | Befestigungsnoppen |
| 10 | Trichter | 421 | Grundkörper |
| 11 | Stutzen | 422 | Verstärkungsnoppen |
| | | 423 | Fahne |
| 2 | Adapter | | |
| 20 | Stutzen | 5 | Durchflussdetektor |
| 21 | Gewindeteil | 50 | Lichtemitter |
| | | 51 | Lichtdetektor |
| 3 | Milchsammelbehälter | 52 | Auswerteelektronik |
| 30 | Flaschenhals | 53 | Verbindungsleitung |
| 4 | Rückschlagventil | 6 | Vakuumpumpe |
| 40 | Ventilsitz | | |
| 40' | Ventilsitz | 7 | Saugleitung |
| 400 | Durchflussöffnung | | |
| 401 | Durchflussöffnung | T | Milchtropfen |
| 402 | Befestigungsöffnung | | |
| 403 | Durchflussöffnung | L | Lichtpfad |
| 404 | Steg | | |
| 41 | Kragen | K | Durchflusskanal |
| 42 | Ventilmembran | | |

## Patentansprüche

1. Vorrichtung mit einem Durchflusskanal (K) für ein Fluid, wobei im Durchflusskanal (K) ein Rückschlagventil (4) angeordnet ist, welches es dem Fluid ermöglicht, den Durchflusskanal (K) in einer ersten Richtung zu durchströmen und welches ein Durchströmen des Durchflusskanals (K) in einer zur ersten Richtung entgegen gesetzten Richtung verhindert, wobei die Vorrichtung ferner einen Durchflussdetektor (5) zur Detektion des Durchflusses des Fluids durch den Durchflusskanal (K) aufweist, wobei der Durchflussdetektor (5) eine Veränderung des Rückschlagventils (4) detektiert, wobei diese detektierte Veränderung als Indikator für den Durchfluss des Fluids dient, wobei das Rückschlagventil (4) eine Ventilmembran (42) aufweist und wobei der Durchflussdetektor (5) eine Auslenkung der Ventilmembran (42) feststellt, **dadurch gekennzeichnet, dass** der Durchflussdetektor (5) einen optischen Detektor zur Detektion der Veränderung des Rückschlagventils (4) aufweist, dass das Rückschlagventil (4) mit einer Fahne (423) versehen ist, deren Lage der Durchflussdetektor (5) detektiert, wobei die Fahne (423) auf der Ventilmembran (42) angeordnet ist, dass der optische Detektor einen Lichtemitter (50) und einen Lichtdetektor (51) umfasst, wobei das Rückschlagventil (4) im veränderten und/oder im unveränderten Zustand in einem Lichtpfad (L) zwischen Lichtemitter (50) und Lichtdetektor (51) angeordnet ist, und dass die Fahne (423) im Lichtpfad (L) angeordnet ist.

2. Vorrichtung nach Anspruch 1, wobei der Durchflussdetektor (5) ein Durchflussmesser zur Bestimmung des Durchflusses ist und wobei die detektierte Veränderung als Mass für den Durchfluss dient.

3. Vorrichtung nach einem der Ansprüche 1 bis 2, wobei die Ventilmembran (42) eine Ventilklappe aufweist.

4. Vorrichtung nach Anspruch 3, wobei die Ventilklappe einen Ventilsitz (40) des Rückschlagventils (4) öffnet und verschliesst, wobei die Ventilklappe in einem ersten Randbereich am Ventilsitz (40) befestigt und an einem zweiten, dem ersten Randbereich gegenüber liegenden Randbereich relativ zum Ventilsitz (40) bewegbar ist.

5. Vorrichtung nach einem der Ansprüche 1 bis 4, wobei die Ventilmembran (42) plan ausgebildet ist.

6. Vorrichtung nach einem der Ansprüche 1 bis 5, wobei die Fahne (423) einem Grundkörper (421) der Ventilmembran (42) senkrecht vorsteht.

7. Vorrichtung nach Anspruch 4, wobei die Fahne (423) im zweiten Randbereich angeordnet ist.

8. Vorrichtung nach einem der Ansprüche 1 bis 7, wobei die Vorrichtung eine Brusthaube (1) einer Brustpumpeneinheit zum Abpumpen menschlicher Muttermilch ist, wobei die Brusthaube (1) zur Anlage auf eine menschliche Mutterbrust ausgebildet ist.

9. Vorrichtung nach einem der Ansprüche 1 bis 7, wobei die Vorrichtung ein Adapter (2) zur Verbindung mit einem Fluidsammelbehälter (3) ist.

10. Vorrichtung nach einem der Ansprüche 1 bis 7, wobei die Vorrichtung Teil einer Drainagevorrichtung, insbesondere Teil eines Fluidsammelbehälters, ist.

## Claims

1. Device having a flow channel (K) for a fluid, wherein a nonreturn valve (4) is arranged in the flow channel (K), said nonreturn valve (4) allowing the fluid to flow through the flow channel (K) in a first direction and preventing it from flowing through the flow channel (K) in a direction counter to the first direction, wherein the device furthermore has a flow detector (5) for detecting the flow of the fluid through the flow channel (K), wherein the flow detector (5) detects a change in the nonreturn valve (4), wherein this detected change serves as an indicator for the flow of the fluid, wherein wherein the nonreturn valve (4) has a valve diaphragm (42) and wherein the flow detector (5) detects a deflection of the valve diaphragm (42), **characterized in that** the flow detector (5) has an optical detector for detecting the change in the nonreturn valve (4), that the nonreturn valve (4) is provided with a lug (423), the position of which is detected by the flow detector (5), wherein the lug (423) is arranged on the valve diaphragm (42), that the optical detector comprises a light emitter (50) and a light detector (51), wherein the nonreturn valve (4) is arranged, in the changed and/or in the unchanged state, in a light path (L) between the light emitter (50) and the light detector (51) and that the lug (423) is arranged in the light path (L).

2. Device according to Claim 1, wherein the flow detector (5) is a flow meter for determining the flow and wherein the detected change serves as a measure of the flow.

3. Device according to one of Claims 1 to 2, wherein the valve diaphragm (42) has a valve flap.

4. Device according to Claim 3, wherein the valve flap opens and closes a valve seat (40) of the nonreturn valve (4), wherein the valve flap is fastened to the valve seat (40) in a first peripheral region and is movable relative to the valve seat (40) in a second peripheral region located opposite the first peripheral region.

5. Device according to one of Claims 1 to 4, wherein the valve diaphragm (42) is formed in a planar manner.

6. Device according to Claim 1 to 5, wherein the lug (423) protrudes perpendicularly from a main body (421) of the valve diaphragm (42).

7. Device according to Claim 4, wherein the lug (423) is arranged in the second peripheral region.

8. Device according to one of Claims 1 to 7, wherein the device is a breast shield (1) of a breastpump unit for expressing human breast milk, wherein the breast shield (1) is designed to be placed on a human breast.

9. Device according to one of Claims 1 to 7, wherein the device is an adapter (2) for connecting to a fluid collection container (3).

10. Device according to one of Claims 1 to 7, wherein the device is part of a drainage device, in particular part of a fluid collection container.

## Revendications

1. Dispositif comprenant un conduit d'écoulement (K) pour un fluide, dans lequel un clapet antiretour (4) est disposé dans le conduit d'écoulement (K), lequel permet au fluide de s'écouler dans une première direction à travers le conduit d'écoulement (K) et empêche un écoulement à travers le conduit d'écoulement (K) dans une direction opposée à la première direction, le dispositif présentant en outre un détecteur d'écoulement (5) pour détecter l'écoulement du fluide à travers le conduit d'écoulement (K), le détecteur d'écoulement (5) détectant une variation du clapet antiretour (4), cette variation détectée servant d'indicateur pour l'écoulement du fluide, le clapet antiretour (4) présentant une membrane de soupape (42) et le détecteur d'écoulement (5) constatant une déviation de la membrane de soupape (42), **caractérisé en ce que** le détecteur d'écoulement (5) présente un détecteur optique pour la détection de la variation du clapet antiretour (4), **en ce que** le clapet antiretour (4) est pourvu d'un fanion (423) dont la position est détectée par le détecteur d'écoulement (5), le fanion (423) étant disposé sur la membrane de soupape (42), **en ce que** le détecteur optique comprend un émetteur de lumière (50) et un détecteur de lumière (51), le clapet antiretour (4) étant disposé dans l'état varié et/ou dans l'état non varié dans un chemin de lumière (L) entre l'émetteur de lumière (50) et le détecteur de lumière (51) et **en ce que** le fanion (423) est disposé dans le chemin de lumière (L).

2. Dispositif selon la revendication 1, dans lequel le détecteur d'écoulement (5) est un débitmètre pour déterminer le débit et la variation détectée sert de mesure pour le débit.

3. Dispositif selon l'une quelconque des revendications 1 et 2, dans lequel la membrane de soupape (42) présente un clapet de soupape.

4. Dispositif selon la revendication 3, dans lequel le clapet de soupape ouvre et ferme un siège de soupape (40) du clapet antiretour (4), le clapet de soupape étant fixé au siège de soupape (40) dans une première région de bord, et pouvant être déplacé par rapport au siège de soupape (40) au niveau d'une deuxième région de bord opposée à la première région de bord.

5. Dispositif selon l'une quelconque des revendications 1 à 4, dans lequel la membrane de soupape (42) est réalisée sous forme plane.

6. Dispositif selon l'une quelconque des revendications 1 à 5, dans lequel le fanion (423) fait saillie perpendiculairement à un corps de base (421) de la membrane de soupape (42).

7. Dispositif selon la revendication 4, dans lequel le fanion (423) est disposé dans la deuxième région de bord.

8. Dispositif selon l'une quelconque des revendications 1 à 7, le dispositif étant une téterelle (1) d'une unité de tire-lait pour pomper du lait maternel humain, la téterelle (1) étant réalisée pour s'appliquer contre un sein maternel humain.

9. Dispositif selon l'une quelconque des revendications 1 à 7, le dispositif étant un adaptateur (2) devant être raccordé à un récipient de collecte de fluide (3).

10. Dispositif selon l'une quelconque des revendications 1 à 7, le dispositif faisant partie d'un dispositif de drainage, en particulier faisant partie d'un récipient de collecte de fluide.
